# EUROPEAN PATENT SPECIFICATION

(11) **EP 4 565 133 B1**
(45) Date of publication and mention of the grant of the patent: **23.09.2026**
(21) Application number: 23761660.2
(22) Date of filing: 03.08.2023
(51) Int. Cl.: A61B 5/1455, A61B 5/0205, A61B 5/021, A61B 5/024, A61B 5/00

(54) **SYSTEM AND METHOD FOR ACCOUNTING FOR A CONFOUNDING FACTOR IN THE DETERMINATION OF A PHYSIOLOGIC PARAMETER OR CONDITION**
SYSTEM UND VERFAHREN ZUR BERÜCKSICHTIGUNG EINES KONDULING-FAKTORS BEI DER BESTIMMUNG EINES PHYSIOLOGISCHEN PARAMETERS ODER EINER PHYSIOLOGISCHEN BEDINGUNG
SYSTÈME ET PROCÉDÉ DE PRISE EN COMPTE D'UN FACTEUR DE CONFUSION DANS LA DÉTERMINATION D'UN PARAMÈTRE OU D'UN ÉTAT PHYSIOLOGIQUE

(30) Priority: 05.08.2022 US 202263370634 P
(43) Date of publication of application: 11.06.2025
(73) Proprietor: Becton, Dickinson and Company, Franklin Lakes, NJ 07417-1880 (US)
(72) Inventor: BENNI, Paul, B., Franklin Lakes, NJ 07417 (US)
(74) Representative: Venner, Julia Ann
(86) International application number: PCT/US2023/029404
(87) International publication number: WO 2024/030548

(56) References cited:
- WO-A1-2022/245530
- US-A1- 2012 197 622
- US-A1- 2012 203 077

## Description

### BACKGROUND OF THE INVENTION

### 1. Technical Field

The present disclosure relates to medical apparatus and methods in general, and to medical apparatus and methods for accounting for a confounding factor in the determination of physiologic parameter or condition in particular.

### 2. Background Information

The determination of a physiologic parameter or condition often relies upon the determination of other physiologic parameters. Very often, physiologic parameters are subject to unrelated influences (referred to herein as a "confounding factor"). For example, a first physiologic parameter may be elevated or depressed because of the influence of a confounding factor. Using that first physiologic parameter to determine a second physiologic parameter may detrimentally influence or taint the determination of the second physiologic parameter; e.g., making the determination less accurate as a result of the influence of the confounding factor. Autoregulation state is an example of a physiologic condition that may be influenced by factors independent of the subject's autoregulation system. A plurality of factors (e.g., a hardening of the arteries that occurs with advancing age) can change the characteristics of a vascular reactivity response, and these factors can in turn change relevant autoregulation characteristics. Hence, the autoregulation range of blood flow due to changing blood pressure can vary between subjects and cannot assumed to be a constant. Furthermore, physiologic parameter data that is used to determine or measure a subject's autoregulation state may be influenced by factors independent of the subject's autoregulation system. For example, one or more NIRS indices (e.g., tissue oxygen saturation (StO₂), relative total hemoglobin concentration per volume of tissue (rTHb), differential changes in oxyhemoglobin (O2Hb) and deoxyhemoglobin (HHb), HbD (i.e., O2Hb - HHb), etc.) may be at a level that is not attributable to autoregulation. In these instances, an autoregulation determination or measurement made using these values may negatively affect the accuracy of the autoregulation determination or measurement. As another example, if a subject's blood carbon dioxide level is outside of a normal range (normocapnia), the accuracy of an autoregulation determination or measurement may be negatively affected.

WO 2022/245530, which forms part of the prior art according to Article 54(3) EPC, describes a method and apparatus for determining a subject's autoregulation function state. The method includes: continuously sensing a tissue region of a subject with a tissue oximeter to produce first signals representative of at least one tissue oxygenation parameter during a period of time; continuously measuring a blood pressure level of the subject during the period of time to produce second signals representative of the subject's blood pressure during the period of time; determining a presence of a confounding factor that affects the sensed tissue oxygenation parameter in a manner independent of an autoregulation function of the subject, the determination using the first signals; using the first and second signals to determine an autoregulation function state of the subject when the absence of the confounding factor is determined. US 2011/13166388 A relates to a method and monitor for monitoring vital signs. In one embodiment, the vital signs monitor includes a plurality of vital signs sensing modules in communication with a processor. The plurality of sensing modules includes at least two of the modules selected from the group of a ballistocardiographic (BCG) module, a photoplethysmographic (PPG) module, an accelerometer module, a temperature measurement module, and an electrocardiographic (ECG) module. In one embodiment, the processor calculates additional vital signs in response to signals from the plurality of vital signs sensing modules. The processor determines if the signal to noise ratio (S/N) of the waveforms provided by the modules is sufficient and if not the data is discarded and additional data collected.

What is needed is an apparatus and method that accounts for one or more confounding factors during the determination of a physiologic parameter or condition.

### SUMMARY

Aspects of the presently claimed invention are set out in the independent claims. Particular embodiments of these aspects are set out in the dependent claims. Any subject matter contained herein that does not fall within the scope of the appended claims is considered as being useful for understanding the invention.

According to an aspect of the present disclosure, a method for determining a target physiologic parameter of a subject is provided. The method includes: a) sensing a subject with a first sensing device configured to sense a first physiologic parameter, the first sensing device producing first physiologic data signals representative of the first physiologic parameter during a period of time; b) sensing the subject with a second sensing device configured to sense a second physiologic parameter, the second sensing device producing second physiologic data signals representative of the second physiologic parameter during the period of time; c) sensing the subject with a third sensing device configured to sense a target physiologic parameter, the third sensing device producing target physiologic data signals representative of the target physiologic parameter during the period of time; d) determining a presence or an absence of a confounding factor that taints a determination of the target physiologic parameter, the determination using the first physiologic data signals, the second physiologic data signals, and the target physiologic data signals; e) advancing the first physiologic data signals, the second physiologic data signals, and the target physiologic data signals produced in the absence of the confounding factor for further processing, and setting aside the first physiologic data signals, the second physiologic data signals, and the target physiologic data signals produced in the presence of the confounding factor; and f) determining a value of the target physiologic parameter using the first physiologic data signals, the second physiologic data signals, and the target physiologic data signals produced in the absence of the confounding factor.

In any of the aspects or embodiments described above and herein, the first physiologic data signals, the second physiologic data signals, and the target physiologic data signals produced in the presence of the confounding factor are not used in the step of determining the value of the target physiologic parameter.

In the presently claimed invention, the step of determining the presence or the absence of the confounding factor uses a frequency domain methodology.

In the presently claimed invention, the step of determining the presence or the absence of the confounding factor includes determining a first coherence between the first physiologic data signals and the target physiologic data signals, and a second coherence between the second physiologic data signals and the target physiologic data signals.

In any of the aspects or embodiments described above and herein, the first coherence may be based on a single band of frequencies.

In any of the aspects or embodiments described above and herein, the first coherence may be representative of coherence values at different individual frequencies within the single band of frequencies.

In any of the aspects or embodiments described above and herein, the first coherence may be based on a plurality of frequency bands.

In any of the aspects or embodiments described above and herein, the first coherence may be collectively representative of a respective coherence value from each respective frequency band of the plurality of frequency bands.

In any of the aspects or embodiments described above and herein, wherein the method may further include determining a first trend of the first physiologic parameter, a second trend of the second physiologic parameter, and a third trend of the target physiologic parameter, and comparing the first trend, the second trend, and the third trend relative to one another.

In any of the aspects or embodiments described above and herein, the step of determining the presence or the absence of the confounding factor may utilize one or more polarity filters configured to evaluate the first trend, the second trend, and the third trend.

In any of the aspects or embodiments described above and herein, the step of determining the presence or the absence of the confounding factor may use an index table.

In any of the aspects or embodiments described above and herein, the setting aside step may include discarding the first physiologic data signals, the second physiologic data signals, and the target physiologic data signals produced in the presence of the confounding factor.

In any of the aspects or embodiments described above and herein, steps a-f may be performed on a continuous basis during the period of time.

In any of the aspects or embodiments described above and herein, the target physiologic parameter may relate to total hemoglobin concentration per volume of tissue (THb) sensed.

In any of the aspects or embodiments described above and herein, the target physiologic parameter may be relative total hemoglobin concentration per volume of tissue (rTHb) of tissue sensed.

In any of the aspects or embodiments described above and herein, the third sensing device may be a near infrared spectroscopy (NIRS) tissue oximeter.

In any of the aspects or embodiments described above and herein, the first physiologic parameter may relate to a blood pressure of the subject, and the first sensing device is a blood pressure sensing device.

In any of the aspects or embodiments described above and herein, the second physiologic parameter may relate to a heart rate of the subject.

According to an aspect of the present disclosure, a system for determining a target physiologic parameter of a subject is provided. The system includes a first sensing device, a second sensing device, a third sensing device, and a system controller. The first sensing device is configured to sense a first physiologic parameter continuously during a period of time, and to produce first physiologic data signals representative of the first physiologic parameter during the period of time. The second sensing device is configured to sense a second physiologic parameter continuously during the period of time, and to produce second physiologic data signals representative of the second physiologic parameter during the period of time. The third sensing device is configured to sense a target physiologic parameter continuously during the period of time, and to produce target physiologic data signals representative of the target physiologic parameter during the period of time. The system controller is in communication with the first sensing device, the second sensing device, and the target sensing device. The system controller includes at least one processor and a memory device configured to store instructions, the stored instructions when executed cause the system controller to: a) determine a presence or an absence of a confounding factor that taints a determination of the target physiologic parameter, the determination using the first physiologic data signals, the second physiologic data signals, and the target physiologic data signals; b) advance the first physiologic data signals, the second physiologic data signals, and the target physiologic data signals produced in the absence of the confounding factor for further processing, and set aside the first physiologic data signals, the second physiologic data signals, and the target physiologic data signals produced in the presence of the confounding factor; and c) determine a value of the target physiologic parameter using the first physiologic data signals, the second physiologic data signals, and the target physiologic data signals produced in the absence of the confounding factor.

In any of the aspects or embodiments described above and herein, the stored instructions when executed may cause the system controller to determine the value of the target physiologic parameter without using the first physiologic data signals, the second physiologic data signals, and the target physiologic data signals produced in the presence of the confounding factor.

In the presently claimed invention, the stored instructions when executed causes the system controller to determine the presence or the absence of the confounding factor using a frequency domain methodology.

In the presently claimed invention, the stored instructions when executed causes the system controller to determine the presence or the absence of the confounding factor further cause the system controller to determine a first coherence between the first physiologic data signals and the target physiologic data signals, and a second coherence between the second physiologic data signals and the target physiologic data signals.

In any of the aspects or embodiments described above and herein, the stored instructions when executed may cause the system controller to determine a first trend of the first physiologic parameter, a second trend of the second physiologic parameter, and a third trend of the target physiologic parameter, and compare the first trend, the second trend, and the third trend relative to one another.

In any of the aspects or embodiments described above and herein, the stored instructions when executed may cause the system controller to determine the presence or the absence of the confounding factor using one or more polarity filters configured to evaluate the first trend, the second trend, and the third trend.

In any of the aspects or embodiments described above and herein, the stored instructions when executed may cause the system controller to determine the presence or the absence of the confounding factor using an index table.

In any of the aspects or embodiments described above and herein, the first physiologic data signals, the second physiologic data signals, and the target physiologic data signals produced in the presence of the confounding factor that are set aside may be discarded.

In any of the aspects or embodiments described above and herein, the stored instructions when executed may cause the system controller to perform functions a-c on a continuous basis during the period of time.

According to another aspect of the present disclosure, a method for determining a target physiologic parameter of a subject is provided that includes the steps of: a) sensing a subject for "N" number of physiologic parameters, where "N" is an integer equal to or greater than three, the sensing producing "N" sets of physiologic data signals, and each physiologic data signal set corresponding to a respective one of the "N" physiologic parameters during a period of time, and wherein one of the "N" number of physiologic parameters is a target physiologic parameter; b) determining a presence or an absence of a confounding factor that taints a determination of the target physiologic parameter, the determination using the "N" sets of physiologic data signals including the target physiologic parameter set of physiologic data signals; c) advancing the "N" sets of physiologic data signals produced in the absence of the confounding factor for further processing, and setting aside the "N" sets of physiologic data signals produced in the presence of the confounding factor; and d) determining a value of the target physiologic parameter using the "N" sets of physiologic data signals produced in the absence of the confounding factor.

According to another aspect of the present disclosure, a non-transitory computer readable medium is provided, the medium storing executable instructions that when executed cause at least one processor to: a) control a first sensing device configured to sense a first physiologic parameter to sense a subject and to produce first physiologic data signals representative of the first physiologic parameter during a period of time; b) control a second sensing device configured to sense a second physiologic parameter to sense the subject and to produce second physiologic data signals representative of the second physiologic parameter during the period of time; c) control a third sensing device configured to sense a target physiologic parameter to sense the subject and to produce target physiologic data signals representative of the target physiologic parameter during the period of time; d) determine a presence or an absence of a confounding factor that taints a determination of the target physiologic parameter, the determination using the first physiologic data signals, the second physiologic data signals, and the target physiologic data signals; e) advance the first physiologic data signals, the second physiologic data signals, and the target physiologic data signals produced in the absence of the confounding factor for further processing, and set aside the first physiologic data signals, the second physiologic data signals, and the target physiologic data signals produced in the presence of the confounding factor; and f) determine a value of the target physiologic parameter using the first physiologic data signals, the second physiologic data signals, and the target physiologic data signals produced in the absence of the confounding factor. In the presently claimed invention, the step of determining the presence or the absence of the confounding factor uses a frequency domain methodology and the step of determining the presence or the absence of the confounding factor includes determining a first coherence between the first physiologic data signals and the target physiologic data signals, and a second coherence between the second physiologic data signals and the target physiologic data signals.

The foregoing features and elements may be combined in various combinations without exclusivity, unless expressly indicated otherwise. These features and elements as well as the operation thereof will become more apparent in light of the following description and the accompanying drawings. It should be understood, however, the following description and drawings are intended to be exemplary in nature and non-limiting.

### BRIEF DESCRIPTION OF THE DRAWINGS

FIG. 1 is a diagrammatic representation of an autoregulation system according to an embodiment of the present disclosure.
FIG. 2 is a diagrammatic representation of an autoregulation system according to an embodiment of the present disclosure.
FIG. 3 is a diagrammatic representation of an exemplary frequency domain method.
FIG. 4 is a diagrammatic flow chart of a present disclosure embodiment.
FIG. 5 is an exemplary index table embodiment that may be used with the present disclosure, illustrating exemplary data from a number of different cases.
FIG. 6 is a diagrammatic flow chart of a present disclosure embodiment.
FIG. 7 is a diagrammatic flow chart of a present disclosure embodiment.
FIG. 8 is a diagrammatic flow chart of a present disclosure embodiment.
FIG. 9 is a diagrammatic flow chart of a present disclosure embodiment.
FIG. 10 is an exemplary index table embodiment that may be used with the present disclosure, illustrating exemplary data from a number of different cases.
FIG. 11 is a diagrammatic graph of physiologic parameter value versus time.
FIG. 12 is a graph of physiologic parameter value versus time.

### DETAILED DESCRIPTION

The present disclosure provides a system 20 ("CFA system"), method, and computer readable medium that accounts for one or more confounding factors during the determination of a physiologic parameter. Nonlimiting examples of physiological parameters that may be determined using the present disclosure include NIRS indices as defined herein, autoregulation status, pain, and others. Embodiments of the present disclosure may be implemented in a variety of different ways. In some embodiments, the present disclosure may be implemented to determine whether one or more confounding factors that may negatively influence the determination of a physiologic parameter are present and if so, account for sensed data collected when the one or more confounding factors are present. In this manner, a physiologic parameter can be determined untainted by a confounding factor. Non-limiting examples of a physiologic parameter determination that may be affected by one or more confounding factors include the determination of different types of total hemoglobin content, the determination of a subject's autoregulation state, and others. The term "confounding factor" as used herein refers to a physiologic parameter or condition that may, when present, influence or taint the determination of another physiologic parameter in a manner that negatively affects the determination of the other physiologic parameter; e.g., the determination of a physiologic parameter that is susceptible to influence from a confounding factor may be less accurate as a result of the influence of the confounding factor. The present disclosure is configured to account for confounding factors when present and thereby mitigate any influence they may otherwise have on the aforesaid determination, thereby leading to an improvement in physiologic parameter determination.

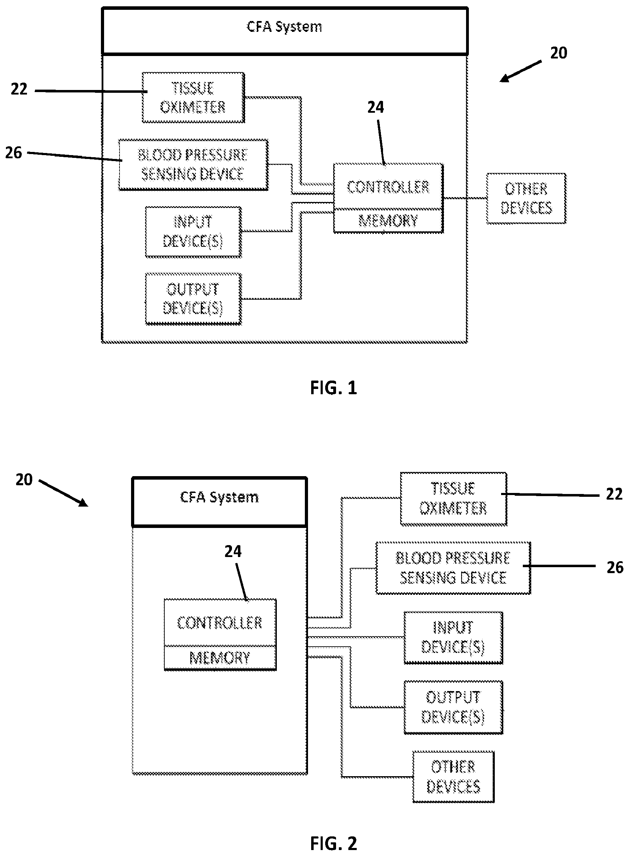

FIGS. 1 and 2 diagrammatically illustrate nonlimiting examples of present disclosure system 20 embodiments, including their respective system components. The system 20 embodiments shown are not intended to be limiting; e.g., alternative system 20 embodiments may include additional components, or alternative components, or different component implementations, and the like. In some embodiments, the system 20 includes system components such as a tissue oximeter 22 and a system controller 24, and may include other system components such as a blood pressure sensing device 26, a carbon dioxide (CO₂) sensor (e.g., a transcutaneous blood gas monitor, or an exhaled bread CO₂ sensor, or the like), a heart rate monitor (e.g., an electrocardiogram - "ECG", etc.), devices configured to sense hemodynamic parameters such as vasoreactivity, cardiac output, blood flow, and the like, one or more output devices, and one or more input devices. In some embodiments, these system components may be integrated into a single system 20 device; e.g., a system controller 24 integrally connected with sensing hardware (e.g., hardware associated with a tissue oximeter 22, hardware associated with a BP sensing device 26, etc.). In other embodiments, the system 20 may include a system controller 24, and may be configured to communicate with (e.g., receive signal data from and/or send signal data to) independent system components. In other words, in embodiments wherein the system 20 includes independent system components, the system 20 may be configured to communicate with a tissue oximeter 22 that is capable of functioning independently of the system 20, with a BP sensing device 26 that is capable of functioning independently of the system 20, with a CO₂ sensor that is capable of functioning independently of the system 20, a heart rate monitor that is capable of functioning independently of the system 20, etc. In other embodiments, the system 20 may include some combination of these system components in integral and independent form. In those embodiments wherein one or more of the aforesaid system components is independent of the system 20, that independent system component may be in communication with the system controller 24 in any manner.

The tissue oximeter 22 may be a device configured to continuously sense a tissue oxygenation parameter (which parameter may be referred to hereinafter individually as a "NIRS index" or collectively as "NIRS indices") that varies with blood flow in a subject's tissue; e.g., tissue oxygen saturation (StO₂), total hemoglobin concentration per volume of tissue (THb), relative total hemoglobin concentration per volume of tissue (rTHb), deoxyhemoglobin (HHb), relative deoxyhemoglobin (rHHb), oxyhemoglobin (O2Hb), relative oxyhemoglobin (rO2Hb), deoxyhemoglobin (HHb), and the like. To be clear, the present disclosure is not limited to these particular NIRS indices and the various acronyms used herein (e.g., StO₂, THb, rTHb, HHb, rHHb, O2Hb, and rO2Hb) are non-limited examples of acronyms that may be used by those skilled in the art to refer to the respective NIRS index. A person of skill in the art will recognize that the same NIRS indices are sometimes referred to using different acronyms.

An example of an acceptable tissue oximeter 22 is a near infra-red spectroscopy ("NIRS") type tissue oximeter ("NIRS tissue oximeter"). U.S. Patent Nos. 6,456,862; 7,072,701; 8,078,250; 8,396,526; and 8,965,472; and 10,117,610, disclose non-limiting examples of a non-invasive NIRS tissue oximeter 22 that may be used within the present disclosure. The term "continuously" as used herein (to describe a tissue oximeter 22 continuously sensing a tissue oxygenation parameter) means that the tissue oximeter 22 senses and collects subject data on a periodic basis during the monitoring time period, which periodic basis is sufficiently frequent that it may be considered to be clinically continuous. For example, some tissue oximeters sample data every ten seconds or less and can be configured to sample data more frequently (e.g., every two seconds or less).

The tissue oximeter 22 includes one or more sensors in communication with a controller portion. Each sensor includes one or more light sources (e.g., light emitting diodes, or "LEDs") and one or more light detectors (e.g., photodiodes, etc.). The light sources are configured to emit light at different wavelengths of light, e.g., wavelengths of light in the red or near infrared range; 400-1000nm. In some sensor embodiments, a sensor may be configured to include a light source, a near detector(s), and a far detector(s). The near detector(s) are disposed closer to the light source than the far detector(s). A non-limiting example of such a sensor is disclosed in U.S. Patent No. 8,965,472. The tissue oximeter 22 is configured for communication with the system controller 24; e.g., send signals representative of (or signals that can be used to determine) one or more NIRS indices to the system controller 24, and may receive control signals, etc. from the system controller 24. Communications between the tissue oximeter 22 and the system controller 24 may be by any known means; e.g., hardwire, wireless, etc.

The NIRS tissue oximeter 22 may utilize one or more algorithms for determining one or more of the NIRS indices. The present disclosure is not limited to any particular NIRS tissue oximeter 22 or any algorithm for determining a NIRS Index of the sensed tissue. U.S. Patent Nos. 9,913,601; 9,848,808; 9,456,773; 9,364,175; 9,923,943; 8,788,004; 8,396,526; 8,078,250; 7,072,701; and 6,456,862 all describe non-limiting examples of algorithms for determining NIRS indices that may be used totally or in part with the present disclosure.

The blood pressure sensing device 26 ("BP sensing device 26") may be any sensor or device configured to continuously determine a subject's blood pressure (e.g., arterial blood pressure). For example, the BP sensing device 26 may be a device that is configured to provide continuous blood pressure measurement, such as an arterial catheter line, or a continuous non-invasive blood pressure device, or a pulse oximetry sensor. The present disclosure is not, however, limited to using these particular examples of blood pressure sensing / measuring / monitoring devices. The BP sensing device 26 is configured to produce blood pressure value signals indicative of the subject's blood pressure (e.g., arterial blood pressure) during a period of time. The BP sensing device 26 is configured for communication with the system controller 24; e.g., send blood pressure value signals to the system controller 24, and may receive control signals, etc. from the system controller 24. Communications between the BP sensing device 26 and the system controller 24 may be by any known means; e.g., hardwire, wireless, etc. The term "continuously" as used herein (to describe a BP sensing device 26 continuously determining a subject's blood pressure) means that the BP sensing device 26 senses and collects subject data on a periodic basis during the monitoring time period, which periodic basis is sufficiently frequent that it may be considered to be clinically continuous. For example, some BP sensing devices sample data every ten seconds or less and can be configured to sample data more frequently (e.g., every two seconds or less).

One or both of the BP sensing device 26 or the tissue oximeter 22 may be further configured to measure other parameters, such as respiratory rate, respiratory effort, heart rate (HR), etc. The BP sensing device 26 and the tissue oximeter 22 may be placed on the same or different parts of the patient's body.

As stated above, the system 20 includes a system controller 24, and may include one or more output devices and one or more input devices. Non-limiting examples of an input device include a keyboard, a touchpad, or other device wherein a user may input data, commands, or signal information, or a port configured for communication with an external input device via hardwire or wireless connection, etc. Non-limiting examples of an output device include any type of display, printer, or other device configured to display or communicate information or data produced by the system 20. The system 20 may be configured for connection with an input device or an output device via a hardwire connection or a wireless connection.

In some embodiments, the system controller 24 may be configured (e.g., via electrical circuitry) to process various received signals (received from integral or independent components) and may be configured to produce certain signals to the same; e.g., signals configured to control one or more components within the system 20. Alternatively, the system 20 may be configured such that signals from the respective component are sent to one or more intermediate processing devices, and the intermediate processing device may in turn provide processed signals or data to the system controller 24. As will be explained below, the system controller 24 may also be configured to execute stored instructions (e.g., algorithmic instructions) that cause the system 20 to perform steps or functions described herein, to produce data (e.g., determine physiologic parameter values in a manner that accounts for one or more confounding factors, etc.), to communicate, etc.

The system controller 24 may include any type of computing device, computational circuit, or any type of process or processing circuit capable of executing a series of instructions that are stored in memory. The controller may include multiple processors and/or multicore CPUs and may include any type of processor, such as a microprocessor, digital signal processor, co-processors, a micro-controller, a microcomputer, a central processing unit, a field programmable gate array, a programmable logic device, a state machine, logic circuitry, analog circuitry, digital circuitry, etc., and any combination thereof. For example, in those embodiments of the system 20 described above that include multiple components (e.g., a BP sensing device 26, a tissue oximeter 22, a CO₂ sensor, etc.) integral with the system 20, the controller may include multiple processors; e.g., an independent processor dedicated to each respective component, any and all of which processors may be in communication with a central processor of the system 20 that coordinates functionality of the system 20. The instructions stored in memory may represent one or more algorithms for controlling the system 20, and the stored instructions are not limited to any particular form (e.g., program files, system data, buffers, drivers, utilities, system programs, etc.) provided they can be executed by the controller. The instructions are configured to perform the methods and functions described herein.

The memory may be a machine readable storage medium configured to store instructions that when executed by one or more processors, cause the one or more processors to perform or cause the performance of certain functions. The memory may be a single memory device or a plurality of memory devices. A memory device may be a non-transitory device and may include a storage area network, network attached storage, as well as a disk drive, a read-only memory, random access memory, volatile memory, non-volatile memory, static memory, dynamic memory, flash memory, cache memory, and/or any device that stores digital information. One skilled in the art will appreciate, based on a review of this disclosure, that the implementation of the controller may be achieved via the use of hardware, software, firmware, or any combination thereof.

Implementation of the techniques, blocks, steps, and means described herein may be done in various ways. For example, these techniques, blocks, steps, and means may be implemented in hardware, software, or a combination thereof. For a hardware implementation, processing devices configured to carry out the described functions and steps (e.g., by executing stored instructions) may be implemented within one or more application specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, or other electronic units designed to perform the functions described herein, and/or any combination thereof.

Embodiments of the present disclosure may be described herein as a process which is depicted as a flowchart, a flow diagram, a block diagram, etc. Although any one of these structures may describe the operations as a sequential process, many of the operations can be performed in parallel and/or concurrently. In addition, the order of the operations may be rearranged. A process may correspond to a method, a function, a procedure, a subroutine, a subprogram, etc.

System components are configured to continuously sense a plurality of physiological parameters (e.g., tissue oximeter data relating to one or more NIRS indices, blood pressure data, heart rate, CO₂ data, etc.) and produce signal data representative thereof in real time. The specific functionality of a system component producing the physiologic signal data (e.g., sampling rate, etc.) can be set as appropriate for the operation of the system 20, and the present disclosure is not limited to any particular component settings.

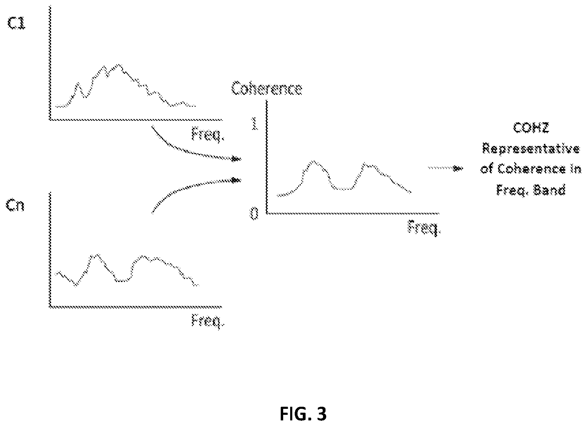

The system 20 may be configured to process this "input" physiologic signal data using an algorithm based on a frequency domain methodology to produce a coherence ("COHZ") analysis, or using an algorithm based on correlation / regression technique, or some combination thereof, as will described in more detailed hereinafter. To facilitate the present description, the present disclosure is described hereinafter utilizing a frequency domain methodology that produces coherence ("COHZ") values, but as indicated the present disclosure is not limited to using a frequency domain methodology. Referring to FIG. 3, the frequency domain methodology transforms (e.g., via a Fourier transformation) pairs of input physiologic parameter signal data from a time domain to a frequency domain. FIG. 3 shows respective plots of a first physiologic parameter ("C1") versus frequency and a second physiologic parameter ("Cn", where "n" is an integer greater than 1) versus frequency. The transformed data is further analyzed to determine the degree of coherence within a single band of frequencies (i.e., a single frequency band). The degree of coherence may be indicated in terms of an arbitrarily assigned scale of zero to one (0 - 1), wherein the degree of coherence increases from zero to one (shown as a plot of coherence values versus frequency). A coherence value of one represents a stronger relationship between C1 and Cn, and a coherence value that approaches zero indicates increasingly less relationship between C1 and Cn. The process of determining the degree of coherence (COHZ) is performed for at least two different physiologic parameter pairs (e.g., C1 and Cn, C2 and Cn) and is not limited to any particular number of physiologic parameter pairs; e.g., the process of determining the degree of coherence (COHZ) may be performed for more than two different physiologic parameter pairs - C1 and Cn, C2 and Cn, C3 and Cn, etc.

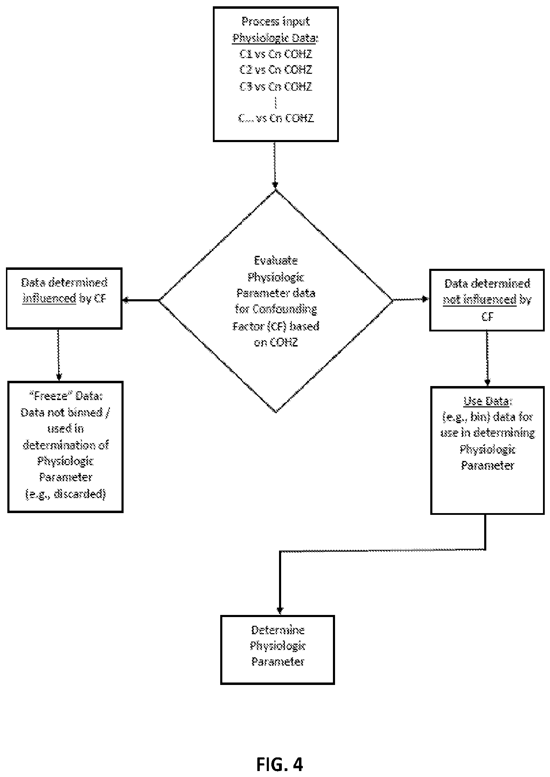

Referring to FIG. 4, the determined coherence values for the respective physiologic parameter pairs are then evaluated to determine whether the collected physiologic paramater data is influenced by a confounding factor. The aforesaid evaluation may be performed in a variety of different ways, including but not limited to using an index table, or a flat filter, or a polarity filter, or the like.

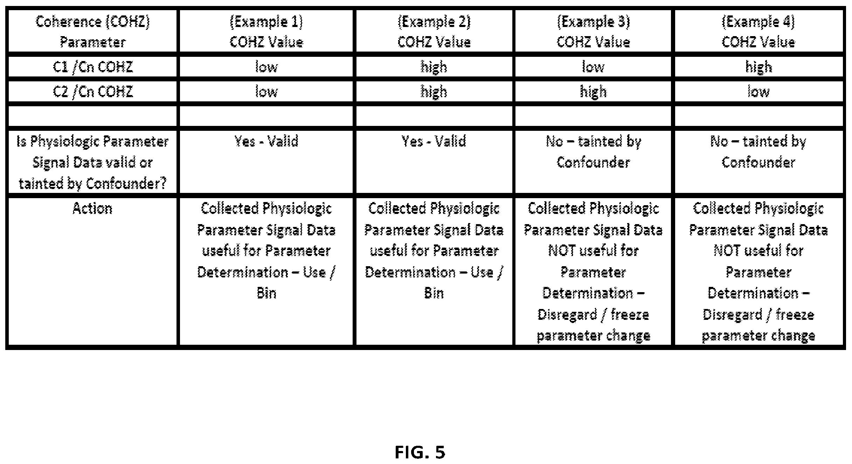

An example of an index table that may be used is one that permits comparison of the coherence value of each physiologic parameter pair relative to the coherence value of another physiologic parameter pair. The index table may contain empirically collected data that permits characterization of the physiologic parameter pair coherence values. The table may assume any data structure form that permits the aforesaid comparison. The table shown in FIG. 5 illustrates four example coherence value scenarios for the C1/Cn physiologic parameter pair and the C2/Cn physiologic parameter pair. In Example 1, both the C1/Cn and the C2/Cn physiologic parameter pairs are indicated as having low coherence values. Instructions stored within memory may include information (e.g., a threshold value) that can be used to determine what is a "low" coherence value, or a "high" coherence value, or an indeterminate coherence value. Based on the empirical data stored within the index table, the agreement between the C1/Cn and the C2/Cn physiologic parameter pair coherence values (i.e., both low) indicates that the physiologic data collected is valid, untainted by a confounding factor, and can be used (e.g., binned or otherwise processed) in a subsequent determination of the physiologic parameter. In Example 2, both the C1/Cn and the C2/Cn physiologic parameter pairs are indicated as having high coherence values. Based on the empirical data stored within the index table, the agreement between the C1/Cn and the C2/Cn physiologic parameter pair coherence values (i.e., both high) indicates that the physiologic data collected is valid, untainted by a confounding factor, and can be used (e.g., binned or otherwise processed) in a subsequent determination of the physiologic parameter. In Examples 3 and 4, the C1/Cn and the C2/Cn physiologic parameter pairs are indicated as having disparate coherence values (i.e., one high, one low). Based on the empirical data stored within the index table, the disagreement between the C1/Cn and the C2/Cn physiologic parameter pair coherence values (i.e., both high) indicates that the physiologic data collected is likely tainted by a confounding factor, and therefore should not be used (e.g., not binned) in a subsequent determination of the physiologic parameter. The collected physiologic data used to determine coherence values in these instances (determined to be confounding factor tainted) may be frozen

(e.g., set aside) or discarded. It should be noted that the present disclosure provides information on a continuous, real time basis. Hence, physiologic data collected during a first period of time may be deemed acceptable and may be used to determine a physiologic parameter, followed a second period of time wherein the physiologic data may be deemed unacceptable and therefore is not used to determine the physiologic parameter. The present disclosure contemplates that the process of distinguishing confounding factor tainted physiologic data from non confounding factor tainted physiologic data may be a real time process that occurs continuously during the sensing period. The stored instructions may cause the confounding factor untainted physiologic data to be binned or otherwise processed into a form (e.g., values representative of a physiologic parameter organized in bins of MAP ranges, etc.) that may be subsequently used to determine a physiologic parameter (e.g., THb, AR, and the like) as a function of time. In this manner, the present disclosure facilitates a real time determination of a physiologic parameter based on confounding factor non-tainted data. As shown in FIG. 4, physiologic data tainted by one or more confounding factors is not binned or otherwise processed, and therefore may not be used to determine a physiologic parameter in the same manner as the untainted physiologic data. In some instances, the tainted physiologic data - now frozen (e.g., set aside) relative to the normal process of determining the physiologic parameter - may be discarded, but discarding the tainted physiologic data is not required. The tainted physiologic data is not used in the real time determination of the physiologic parameter.

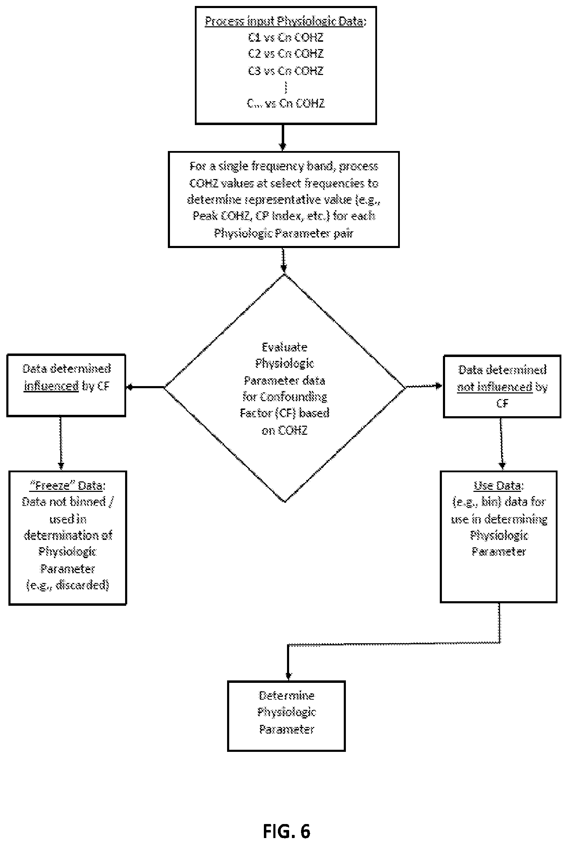

Referring to FIG. 6, in the determination of a coherence value within a single band of frequencies between two physiologic parameters, the coherence value may vary as a function of the particular frequencies within the frequency band; e.g. see FIG. 3. In some embodiments, the present disclosure may operate to select a value that is representative of the coherence values at different frequencies within the frequency band. For example, in some embodiments the present disclosure may operate to select a peak coherence value (i.e., greatest magnitude) from the coherence values at the respective frequencies within the frequency band as a representative coherence value. That peak coherence value may then be subsequently used for purposes of determining the acceptablility of the physiologic parameter signal data for determining a physologic parameter value. In some embodiments, select coherence values within the single band of frequencies may be processed to produce a collective coherence parameter (referred to hereinafter as "CP Index") representative of the coherence values within the band of frequencies. The CP Index may be used subsequently for purposes of determining the acceptablility of the physiologic parameter signal data for determining a physiologic parameter value. A first example of how a CP Index value may be determined is an averaging (or process similar to averaging) of the coherence values at the select frequencies within the frequency band. A second example of how a CP Index value may be determined is a process wherein the coherence values at the select frequencies within the frequency band are multiplied and the square root of the multiplication product is determined to produce the CP Index value. The present disclosure is not limited to these examples of determining a CP Index and a CP Index value may be determined using alternative processes. The present disclosure is also not limited to these examples (i.e., Peak COHZ, CP Index) of selecting a value that is representative of the coherence values at different frequencies within the frequency band.

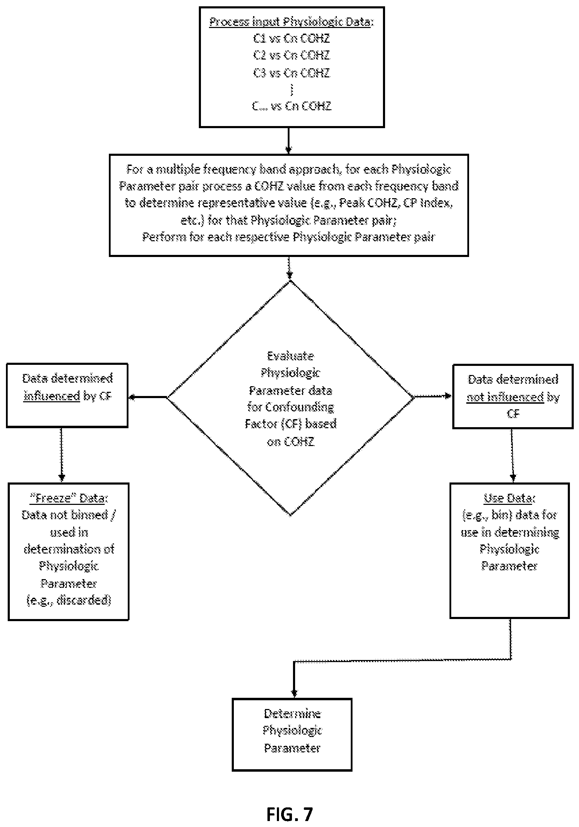

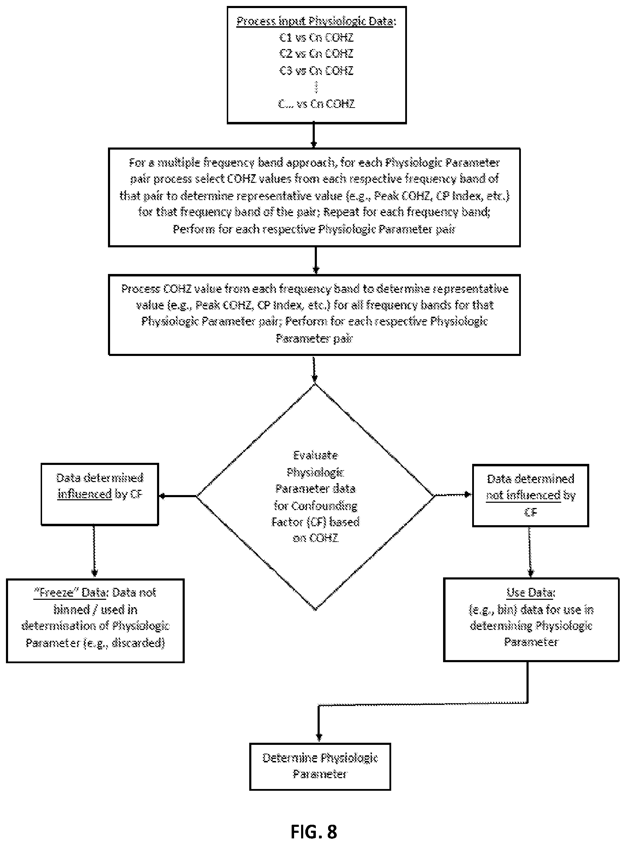

In the above example provided to illustrate embodiments of the present disclosure configured to evaluate physiologic parameter data for the presence of a confounding factor, the coherence value (e.g., peak, CP Index, etc.) of a pair of physiologic parameters (e.g., either C1 and Cn or C2 and Cn) is determined within a single band of frequencies (e.g., see FIG. 3). Referring to FIGS. 7 and 8, in some embodiments coherence values for a given pair of physiologic parameters may be determined within a plurality of different frequency bands. Sensing physiologic parameter data in different frequency bands can, for certain physiologic parameters, produce more robust information regarding that physiologic parameter. For example, a first frequency band with a first duration sampling window may be useful to provide coherence data relating to rapid changes in the physiologic parameter, a second frequency band with a second duration sampling window may be useful to provide coherence data relating to changes in the physiologic parameter that occur less rapidly than those likely to be sensed within the first frequency band (e.g., changes not readily determinable in the first frequency band), and a third frequency band with a third duration sampling window may be useful to provide coherence data relating to changes in the physiologic parameter that occur less rapidly than those likely to be sensed within the second frequency band, etc. Other frequency bands may be selected based on their ability to evaluate a physiologic characteristic of a subject and their ability to identify coherence between that physiologic characteristic and the physiologic parameter under consideration.

In those instances wherein coherence values (e.g., COHZ at a given freq., Peak COHZ, CP Index, etc.) for a given pair of physiologic parameters are determined within each of a plurality of different frequency bands, the determined coherence values for the given pair of physiologic parameters may be further processed to determine a collective coherence value (e.g., a Peak COHZ, CP Index, etc.) based on the determined coherence values from the different frequency bands. As an example (see FIG. 7), selected coherence values from each frequency band for each given physiologic parameter pair may be processed to produce a collective coherence value for all the frequency bands for that physiologic parameter pair in the manner described above (e.g., a Peak COHZ, or a CP Index, etc.).

FIG. 8 illustrates an embodiment utilizing a combination of the processing described above and shown in FIGS. 6 and 7. More specifically, for each respective frequency band of the multiple frequency bands, select coherence values within each of the frequency bands may be processed to produce a collective coherence parameter (e.g., a Peak COHZ, a CP Index, etc.) representative of the coherence values within that frequency band. This process is repeated for each of the frequency bands for each respective physiologic parameter pair; e.g., as described above under FIG. 6. The collective coherence value for each frequency band for a given physiologic pair is subsequently processed in the same manner to provide a collective coherence value (e.g., a Peak COHZ, or a CP Index, etc.) for all of frequency bands for that physiologic pair. This process is performed for each respective physiologic parameter pair, and the respective collective coherence values (e.g., a Peak COHZ, or a CP Index, etc.) are subsequently passed to an evaluation of a confounding factor step.

In some embodiments, the present disclosure may determine coherence values or values representative of coherence values (e.g., Peak COHZ, CP Index, etc.) over a period of time and process those values to determine trend information; e.g., whether a physiologic parameter is trending upward over the period of time, or trending downward over the period of time, or remaining stable over the period of time. In some applications, trending information may be used in the determination of whether collected physiologic parameter data is acceptable and therefore can be used to determine a physiologic parameter, or unacceptable and therefore should not be used to determine a physiologic parameter. For example, a physiologic parameter that is increasing over the period of time may be characterized as being in an increasing trend ("Incr"), a physiologic parameter that is decreasing over the period of time may be characterized as being in an decreasing trend ("Decr"), and a physiologic parameter that is stable over the period of time may be characterized as being stable ("N/C"). Relative trends between different physiologic parameters may provide information regarding the usefulness of collected physiologic parameter data. In some embodiments, trending information may be used within a polarity filter. For example, if a first physiologic parameter is trending upward and a second physiologic parameter is trending downward, the fact that the two physiologic parameters have opposite trending "polarity" (one decreasing, one increasing) may provide the basis for a determination of whether collected physiologic parameter data is acceptable or unacceptable. As another example, if both physiologic parameters are trending in the same direction (i.e., similar polarity), that may provide the basis for a determination of whether collected physiologic parameter data is acceptable or unacceptable. In some embodiments, the present disclosure process (e.g., within the stored instructions) may include a polarity filter that operates to determine whether collected physiologic parameter data is acceptable or unacceptable. Stored instructions may provide a means for determining whether a physiologic parameter is trending upward or downward or remaining stable; e.g., a rate of change threshold, or a change magnitude threshold, or the like.

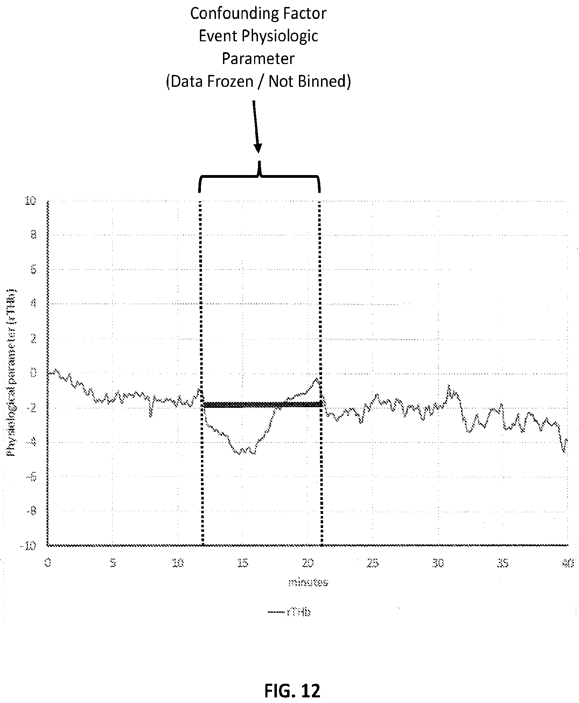

As stated above, the present disclosure is directed to a process of distinguishing physiologic data tainted by a confounding factor from that untainted by a confounding factor, which process may be a real time continuous process. The untainted physiologic data may subsequently be used to determine a physiologic parameter as a function of time. Hence, the present disclosure including the exemplary embodiments disclosed above permits the determination of a physiologic parameter with a greater degree of certainty than would be otherwise possible if confounding factors were not accounted for. The flow charts in FIGS. 4 and 6-9 illustrate the present disclosure on a continuous basis; e.g., the process steps may be continuously executed during the period of time in which the subject is sensed to produce the physiologic data. The sensed physiologic data is processed (e.g., "Process Input Phyiologic Data") and the processed data is subsequently subject to the various additional steps indicated in the respective steps indicated in the respective flow steps, including evaluating the data to determine if tainted by a confounding factor (if so, data is frozen or set aside) or not tainted by a confounding factor (if so, advanced for further processing). The final step shown in the exemplary embodiments ("Determine Phyiologic Parameter" or "Determine THb" - i.e., further processing) can then include displaying the determined values as a function of time; e.g., in a manner similar to that shown in FIG. 12. As stated herein, the present disclosure may be used with a variety of different methodologies for determining a physiologic parameter value and/or for displaying a physiologic parameter value, and is therefore not limited to any particular methodology for determining a physiologic parameter value and/or for displaying a physiologic parameter value.

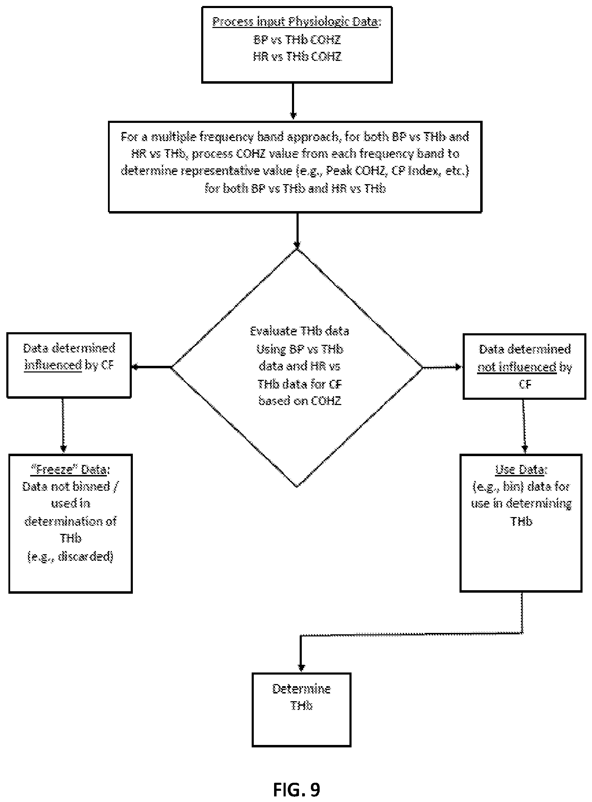

To facilitate an understanding of the scope and utility of the present disclosure, a specific example of the present disclosure is provided hereinafter. This example provides a process shown diagrammatically in FIG. 9 for evaluating physiologic data collected for determining total hemoglobin data (THb) and whether a hemodynamic confounder (e.g., a change in heart rate, a change in BP, hemodilution, etc.) is present that may taint a determination of THb. In this example, three physiologic parameters are sensed (e.g., THb, BP and HR) and two pairs of physiologic parameters are utilized.

In this example, the present disclosure system 20 includes a tissue oximeter 22 configured to continuously produce data representative of a subject's THb, a BP sensing device configured to continuously produce data representative of the subject's blood pressure, and a HR monitor configured to produce data representative of the subject's heart rate. Alternatively, the system 20 may be configured to produce the heart rate data using the tissue oximeter 22 or the BP sensing device. The tissue oximeter 22, BP sensing device, and the HR monitor may be integral to the system 20 or independent devices in communication with a system controller 24.

The system 20 is operated to produce signal data representative of the subject's THb, BP, and HR. The signal data representative of the subject's THb, BP, and HR is processed by the present disclosure system 20 using an algorithm based on a frequency domain methodology to produce a coherence analysis. The frequency domain methodology transforms (e.g., via a Fourier transformation) pairs of input physiologic parameter signal data from a time domain to a frequency domain (e.g., see FIG. 3). In this example, the pairs of physiologic parameter signal data are BP vs THb and HR vs THb. The process subsequently determines a degree of coherence either within a single frequency band or within a plurality of frequency bands as described above. If the system 20 is configured to determine coherence in a single frequency band, then a single coherence value may be determined for the BP vs THb pair and a single coherence value may be determined for the HR vs THb pair. Those coherence values are subsequently evaluated to determine whether the THb data is tainted by a confounding factor. If the system 20 is configured to determine coherence in multiple frequency bands, then a coherence value may be determined for each frequency band and a collective coherence value (e.g., Peak COHZ) or a value collectively representative of the coherence values for the frequency bands (e.g., CP Index) may be determined. The collective coherence values are subsequently evaluated to determine whether the THb data is tainted by a confounding factor.

As described above, the present disclosure system 20 may be configured to evaluate coherence values to determine whether the THb data is tainted by a confounding factor in a variety of different ways. In some embodiments, an index table containing empirically collected data may be used that characterizes the respective coherence values. For example, as described above and shown in FIG. 5 coherence values may be characterized as low, or high, or indeterminate. The index table may be configured to produce an output indicating whether the input physiologic parameter data is tainted by a confounding factor. The output may be binary; i.e., if the input physiologic parameter data (e.g., THb, BP and HR) is determined to be tainted by a confounding factor, the data is either set aside (i.e., frozen) or discarded, or if the input physiologic parameter data is determined to be untainted by a confounding factor, the data is advanced for use in a determination of the subject's THb.

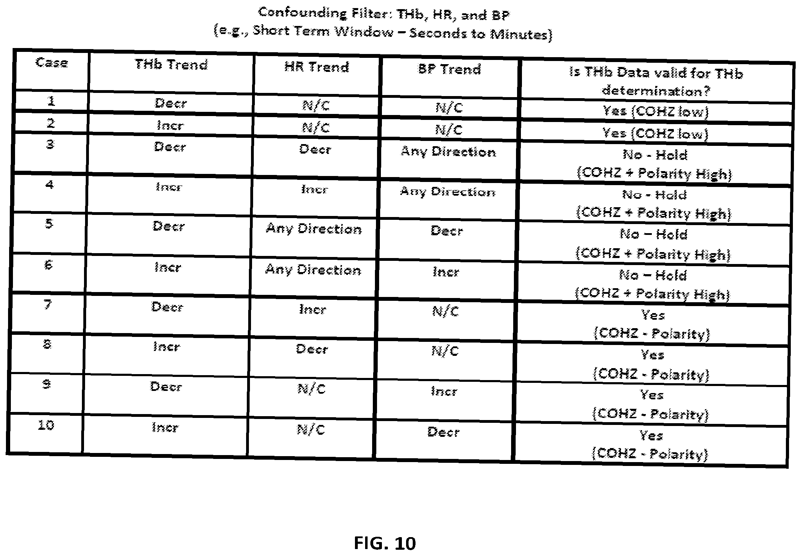

Referring to FIG. 10, as another example the system 20 may be configured to determine the above described coherence values and perform the evaluation over a period of time. Physiologic parameter (e.g., THb, BP and HR) data may be stored and trend data may be developed for each physiologic parameter. The trend data may then be used as a basis for determination of whether the physiologic parameter data of interest (i.e., THb) is tainted by a confounding factor. The input table shown in FIG. 10 illustrates trend data for each physiologic parameter for ten (10) different cases. The input table shown in FIG. 10 includes a binary determination column wherein for each case a determination of whether the physiologic parameter data of interest (i.e., THb) is valid for use is indicated based on physiologic parameter pair coherence and polarity. In cases 1, 2, and 7-10 the input physiologic parameter data (e.g., THb, BP and HR) is determined to untainted by a confounding factor and the data may be passed on for use in a determination of the subject's THb. In cases 3-6, the input physiologic parameter data (e.g., THb, BP and HR) is determined to be tainted by one or more confounding factors and the data is either set aside ("frozen") or discarded and therefore not passed on for use in a determination of the subject's THb.

As stated above, the evaluation of whether the physiologic parameter data of interest (in this example THb) is valid for use in a determination of the physiologic parameter of interest may be performed in a variety of different ways and is not therefore limited to using an index table.

To be clear, the example provided above is given in the context of physiologic parameter data collected for the purpose of producing a THb determination. The present disclosure is not limited to determining evaluating confounding factor influence on physiologic parameter data collected for the purpose of producing a THb determination. On the contrary, the present disclosure may be used to evaluating confounding factor influence on a variety of different physiologic parameters.

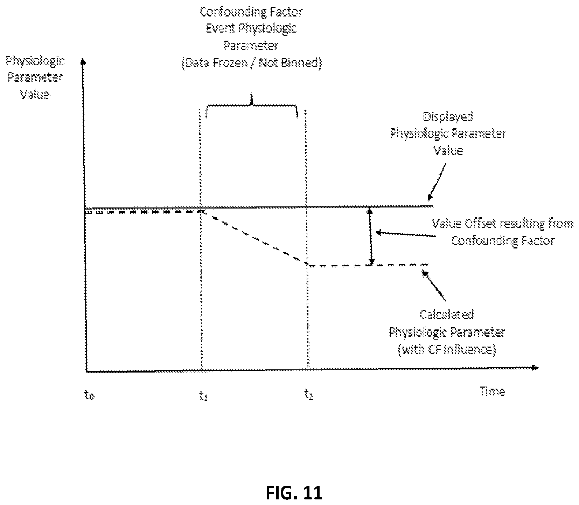

As stated above, the present disclosure is directed to a process of distinguishing physiologic data tainted by a confounding factor from that untainted by a confounding factor (which process may be a real time continuous process), and thereby permitting the determination of a physiologic parameter based solely on untainted physiologic data. In some instances, including sensed physiologic data tainted by a confounding factor in a determination of a physiologic parameter can result in the physiologic parameter value determined being different from the value that would be determined based solely on untainted physiologic data. FIG. 11 diagrammatically illustrates a graph of physiologic parameter value versus time. The graph diagrammatically illustrates two data curves; one solid line, one dashed line. The solid line curve represents a physiologic parameter value based on physiologic data untainted by a confounding factor as a function of time. The dashed line curve represents a physiologic parameter value based on physiologic data unchecked for confounding factors as a function of time. Initially (i.e., from t₀ to t₁) the two data curves are parallel and substantial equal (separated in the graph so they can be seen). Between t₁ and t₂, the physiologic data sensed is tainted by a confounding factor. The confounding factor data tainted causes the determined physiologic parameter to have a different data value than it would if the physiologic parameter value was determined based solely on untainted physiologic data. The different between the tainted and untainted physiologic parameter values is identified on the graph as an offset resulting from the confounding factor. In instances where a physiologic parameter value is determined based on binned physiologic data, the effect of the tainted physiologic sensed data can (if included) have an effect after the period of time in which the confounding factor event occurs; e.g., after t₂.

FIG. 12 is a graph of physiologic parameter value (e.g., THb) versus time. The graph diagrammatically illustrates a confounding factor event that starts at about twelve minutes (12 mins) and extends to about twenty-one minutes (21 mins). As described above, pursuant to the present disclosure the physiologic sensed data may be continuously evaluated to determine the presence of a confounding factor. Upon determining the presence of a confounding factor (e.g., at the 12 minute mark), the sensed physiologic data is frozen / set aside and is not used in any determination of the physiologic parameter (e.g., THb). The displayed data subsequent to the point of "frozen" data (e.g., during the confounding event period), may be displayed in a variety of manners; e.g., the data may be displayed in a manner that indicates the presence of tainted physiologic sensed data; or may be displayed as a constant value based on last untainted physiologic parameter value, or otherwise. The present disclosure is not limited to these display options. Upon determining that the sensed physiologic data is no longer tainted by a confounding factor (e.g., at the 21 minute mark), the system 20 may resume using the sensed physiologic data (now untainted) in a determination of the physiologic parameter and the determined physiologic parameter value can again be displayed.

In some embodiments, the system instructions when executed may cause the controller to use the valid physiologic parameter value just prior to the onset of the confounding factor event (i.e., the 12 minute mark) and the valid physiologic parameter value from just after the confounding factor event concludes (i.e., the 21 minute mark) to determine the displayed value curve between the onset and conclusion of the confounding factor event. For example, the system instructions may determine the difference between physiologic parameter value at the confounding factor onset (i.e., the 12 minute mark) and the physiologic parameter value at the confounding factor conclusion (i.e., the 21 minute mark) and use that difference to produce the displayed value curve between the onset and conclusion of the confounding factor event; e.g., the displayed value may be a straight line or otherwise. The present disclosure is not limited to any particular methodology for displaying a physiologic parameter value determined pursuant to the present disclosure.

While the principles of the disclosure have been described above in connection with specific apparatuses and methods, it is to be clearly understood that this description is made only by way of example and not as limitation on the scope of the disclosure. Specific details are given in the above description to provide a thorough understanding of the embodiments.
However, it is understood that the embodiments may be practiced without these specific details.

It is noted that the embodiments may be described as a process which is depicted as a flowchart, a flow diagram, a block diagram, etc. Although any one of these structures may describe the operations as a sequential process, many of the operations can be performed in parallel or concurrently. In addition, the order of the operations may be rearranged. A process may correspond to a method, a function, a procedure, a subroutine, a subprogram, etc.

The singular forms "a," "an," and "the" refer to one or more than one, unless the context clearly dictates otherwise. For example, the term "comprising a specimen" includes single or plural specimens and is considered equivalent to the phrase "comprising at least one specimen." The term "or" refers to a single element of stated alternative elements or a combination of two or more elements unless the context clearly indicates otherwise. As used herein, "comprises" means "includes." Thus, "comprising A or B," means "including A or B, or A and B," without excluding additional elements.

It is noted that various connections are set forth between elements in the present description and drawings (the contents of which are included in this disclosure by way of reference). It is noted that these connections are general and, unless specified otherwise, may be direct or indirect and that this specification is not intended to be limiting in this respect. Any reference to attached, fixed, connected or the like may include permanent, removable, temporary, partial, full and/or any other possible attachment option.

No element, component, or method step in the present disclosure is intended to be dedicated to the public regardless of whether the element, component, or method step is explicitly recited in the claims. No claim element herein is to be construed under the provisions of 35 U.S.C. 112(f) unless the element is expressly recited using the phrase "means for." As used herein, the terms "comprises", "comprising", or any other variation thereof, are intended to cover a non-exclusive inclusion, such that a process, method, article, or apparatus that comprises a list of elements does not include only those elements but may include other elements not expressly listed or inherent to such process, method, article, or apparatus.

While various inventive aspects, concepts and features of the disclosures may be described and illustrated herein as embodied in combination in the exemplary embodiments, these various aspects, concepts, and features may be used in many alternative embodiments, either individually or in various combinations and sub-combinations thereof. Unless expressly excluded herein all such combinations and sub-combinations are intended to be within the scope of the present application. Still further, while various alternative embodiments as to the various aspects, concepts, and features of the disclosures--such as alternative materials, structures, configurations, methods, devices, and components, and so on--may be described herein, such descriptions are not intended to be a complete or exhaustive list of available alternative embodiments, whether presently known or later developed. Those skilled in the art may readily adopt one or more of the inventive aspects, concepts, or features into additional embodiments and uses within the scope of the present application even if such embodiments are not expressly disclosed herein. For example, in the exemplary embodiments described above within the Detailed Description portion of the present specification, elements may be described as individual units and shown as independent of one another to facilitate the description. In alternative embodiments, such elements may be configured as combined elements. It is further noted that various method or process steps for embodiments of the present disclosure are described herein. The description may present method and/or process steps as a particular sequence. However, to the extent that the method or process does not rely on the particular order of steps set forth herein, the method or process should not be limited to the particular sequence of steps described. As one of ordinary skill in the art would appreciate, other sequences of steps may be possible. Therefore, the particular order of the steps set forth in the description should not be construed as a limitation.

Additionally, even though some features, concepts, or aspects of the disclosures may be described herein as being a preferred arrangement or method, such description is not intended to suggest that such feature is required or necessary unless expressly so stated. Still further, exemplary or representative values and ranges may be included to assist in understanding the present application, however, such values and ranges are not to be construed in a limiting sense and are intended to be critical values or ranges only if so expressly stated.

The treatment techniques, methods, and steps described or suggested herein may be performed on a living animal or on a non-living simulation, such as on a cadaver, cadaver heart, anthropomorphic ghost, or simulator (e.g., with the body parts, or tissue being simulated).

Any of the various systems, devices, apparatuses, etc. in this disclosure may be sterilized (e.g., with heat, radiation, ethylene oxide, hydrogen peroxide) to ensure they are safe for use with patients, and the methods herein may comprise sterilization of the associated system, device, apparatus, etc.; e.g., with heat, radiation, ethylene oxide, hydrogen peroxide.

## Claims

1. A method for determining a target physiologic parameter of a subject, comprising:
a) sensing a subject with a first sensing device configured to sense a first physiologic parameter, the first sensing device producing first physiologic data signals representative of the first physiologic parameter during a period of time;
b) sensing the subject with a second sensing device configured to sense a second physiologic parameter, the second sensing device producing second physiologic data signals representative of the second physiologic parameter during the period of time;
c) sensing the subject with a third sensing device configured to sense a target physiologic parameter, the third sensing device producing target physiologic data signals representative of the target physiologic parameter during the period of time;
d) determining a presence or an absence of a confounding factor that taints a determination of the target physiologic parameter, the determination using the first physiologic data signals, the second physiologic data signals, and the target physiologic data signals, wherein the step of determining the presence or the absence of the confounding factor uses a frequency domain methodology and wherein the step of determining the presence or the absence of the confounding factor includes determining a first coherence between the first physiologic data signals and the target physiologic data signals, and a second coherence between the second physiologic data signals and the target physiologic data signals;
e) advancing the first physiologic data signals, the second physiologic data signals, and the target physiologic data signals produced in the absence of the confounding factor for further processing, and setting aside the first physiologic data signals, the second physiologic data signals, and the target physiologic data signals produced in the presence of the confounding factor; and
f) determining a value of the target physiologic parameter using the first physiologic data signals, the second physiologic data signals, and the target physiologic data signals produced in the absence of the confounding factor.

2. The method of claim 1, wherein the first physiologic data signals, the second physiologic data signals, and the target physiologic data signals produced in the presence of the confounding factor are not used in the step of determining the value of the target physiologic parameter.

3. The method of claim 1 or claim 2, wherein:
a) the first coherence are based on a single band of frequencies, optionally wherein the first coherence is representative of coherence values at different individual frequencies within the single band of frequencies; or
b) the first coherence is based on a plurality of frequency bands, optionally wherein the first coherence is collectively representative of a respective coherence value from each respective frequency band of the plurality of frequency bands.

4. The method of claim 1 or claim 2, further comprising determining a first trend of the first physiologic parameter, a second trend of the second physiologic parameter, and a third trend of the target physiologic parameter, and comparing the first trend, the second trend, and the third trend relative to one another,
optionally wherein the step of determining the presence or the absence of the confounding factor utilizes one or more polarity filters configured to evaluate the first trend, the second trend, and the third trend.

5. The method of any preceding claim, wherein:
a) the step of determining the presence or the absence of the confounding factor uses an index table; and/or
b) steps a-f are performed on a continuous basis during the period of time.

6. The method of any preceding claim, wherein the target physiologic parameter relates to total hemoglobin concentration per volume of tissue (THb) sensed,
optionally wherein the target physiologic parameter is relative total hemoglobin concentration per volume of tissue (rTHb) of tissue sensed, and
optionally wherein the third sensing device is a near infrared spectroscopy (NIRS) tissue oximeter.

7. The method of claim 6, wherein:
a) the first physiologic parameter relates to a blood pressure of the subject, and the first sensing device is a blood pressure sensing device; and/or
b) the second physiologic parameter relates to a heart rate of the subject.

8. A system (20) for determining a target physiologic parameter of a subject, comprising:
a first sensing device (26) configured to sense a first physiologic parameter continuously during a period of time, and to produce first physiologic data signals representative of the first physiologic parameter during the period of time;
a second sensing device configured to sense a second physiologic parameter continuously during the period of time, and to produce second physiologic data signals representative of the second physiologic parameter during the period of time;
a third sensing device (22) configured to sense a target physiologic parameter continuously during the period of time, and to produce target physiologic data signals representative of the target physiologic parameter during the period of time;
a system controller (24) in communication with the first sensing device, the second sensing device, and the target sensing device, the system controller including at least one processor and a memory device configured to store instructions, the stored instructions when executed cause the system controller to:
a) determine a presence or an absence of a confounding factor that taints a determination of the target physiologic parameter, the determination using the first physiologic data signals, the second physiologic data signals, and the target physiologic data signals, wherein the stored instructions when executed cause the system controller to determine the presence or the absence of the confounding factor using a frequency domain methodology, and cause the system controller to determine a first coherence between the first physiologic data signals and the target physiologic data signals, and a second coherence between the second physiologic data signals and the target physiologic data signals;
b) advance the first physiologic data signals, the second physiologic data signals, and the target physiologic data signals produced in the absence of the confounding factor for further processing, and set aside the first physiologic data signals, the second physiologic data signals, and the target physiologic data signals produced in the presence of the confounding factor; and
c) determine a value of the target physiologic parameter using the first physiologic data signals, the second physiologic data signals, and the target physiologic data signals produced in the absence of the confounding factor.

9. The system of claim 8, wherein the stored instructions when executed cause the system controller to determine the value of the target physiologic parameter without using the first physiologic data signals, the second physiologic data signals, and the target physiologic data signals produced in the presence of the confounding factor.

10. The system of claim 8 or claim 9, wherein:
a) the first coherence is based on a single band of frequencies, optionally wherein the first coherence is representative of coherence values at different individual frequencies within the single band of frequencies; or
b) the first coherence is based on a plurality of frequency bands, optionally wherein the first coherence is collectively representative of a respective coherence value from each respective frequency band of the plurality of frequency bands.

11. The system of any of claims 8 to 10, wherein the stored instructions when executed cause the system controller to determine a first trend of the first physiologic parameter, a second trend of the second physiologic parameter, and a third trend of the target physiologic parameter, and compare the first trend, the second trend, and the third trend relative to one another,
optionally wherein the stored instructions when executed cause the system controller to determine the presence or the absence of the confounding factor using one or more polarity filters configured to evaluate the first trend, the second trend, and the third trend.

12. The system of claim 21, wherein the stored instructions when executed cause the system controller to:
a) determine the presence or the absence of the confounding factor using an index table; and/or
b) perform functions a-c on a continuous basis during the period of time.

13. The system of any of claims 8 to 12, wherein the target physiologic parameter relates to total hemoglobin concentration per volume of tissue (THb) sensed,
optionally wherein the target physiologic parameter is relative total hemoglobin concentration per volume of tissue (rTHb) of tissue sensed, and
optionally wherein the third sensing device is a near infrared spectroscopy (NIRS) tissue oximeter.

14. The system of claim 13, wherein:
a) the first physiologic parameter relates to a blood pressure of the subject, and the first sensing device is a blood pressure sensing device; and/or
b) second physiologic parameter relates to a heart rate of the subject.

15. A non-transitory computer readable medium storing executable instructions that when executed cause at least one processor to:
control a first sensing device configured to sense a first physiologic parameter to sense a subject and to produce first physiologic data signals representative of the first physiologic parameter during a period of time;
control a second sensing device configured to sense a second physiologic parameter to sense the subject and to produce second physiologic data signals representative of the second physiologic parameter during the period of time;
control a third sensing device configured to sense a target physiologic parameter to sense the subject and to produce target physiologic data signals representative of the target physiologic parameter during the period of time;
determine a presence or an absence of a confounding factor that taints a determination of the target physiologic parameter, the determination using the first physiologic data signals, the second physiologic data signals, and the target physiologic data signals, wherein the step of determining the presence or the absence of the confounding factor uses a frequency domain methodology and wherein the step of determining the presence or the absence of the confounding factor includes determining a first coherence between the first physiologic data signals and the target physiologic data signals, and a second coherence between the second physiologic data signals and the target physiologic data signals;
advance the first physiologic data signals, the second physiologic data signals, and the target physiologic data signals produced in the absence of the confounding factor for further processing, and set aside the first physiologic data signals, the second physiologic data signals, and the target physiologic data signals produced in the presence of the confounding factor; and
determine a value of the target physiologic parameter using the first physiologic data signals, the second physiologic data signals, and the target physiologic data signals produced in the absence of the confounding factor.

## Patentansprüche

1. Verfahren zum Bestimmen eines physiologischen Zielparameters eines Subjekts, umfassend:
a) Erfassen eines Subjekts mit einer ersten Erfassungsvorrichtung, die dazu ausgelegt ist, einen ersten physiologischen Parameter zu erfassen, wobei die erste Erfassungsvorrichtung erste physiologische Datensignale erzeugt, die repräsentativ für den ersten physiologischen Parameter während eines Zeitraums sind;
b) Erfassen des Subjekts mit einer zweiten Erfassungsvorrichtung, die dazu ausgelegt ist, einen zweiten physiologischen Parameter zu erfassen, wobei die zweite Erfassungsvorrichtung zweite physiologische Datensignale erzeugt, die repräsentativ für den zweiten physiologischen Parameter während des Zeitraums sind;
c) Erfassen des Subjekts mit einer dritten Erfassungsvorrichtung, die dazu ausgelegt ist, einen physiologischen Zielparameter zu erfassen, wobei die dritte Erfassungsvorrichtung physiologische Zieldatensignale erzeugt, die repräsentativ für den physiologischen Zielparameter während des Zeitraums sind;
d) Bestimmen eines Vorliegens oder Nichtvorliegens eines Störfaktors, der eine Bestimmung des physiologischen Zielparameters verfälscht, wobei bei der Bestimmung die ersten physiologischen Datensignale, die zweiten physiologischen Datensignale und die physiologischen Zieldatensignale verwendet werden, wobei in dem Schritt des Bestimmens des Vorliegens oder Nichtvorliegens des Störfaktors eine Frequenzbereichsmethodik verwendet wird und wobei der Schritt des Bestimmens des Vorliegens oder Nichtvorliegens des Störfaktors Bestimmen einer ersten Kohärenz zwischen den ersten physiologischen Datensignalen und den physiologischen Zieldatensignalen und einer zweiten Kohärenz zwischen den zweiten physiologischen Datensignalen und den physiologischen Zieldatensignalen beinhaltet;
e) Weiterleiten der ersten physiologischen Datensignale, der zweiten physiologischen Datensignale und der physiologischen Zieldatensignale, die bei Nichtvorliegen des Störfaktors erzeugt werden, zur weiteren Verarbeitung und Zurückstellen der ersten physiologischen Datensignale, der zweiten physiologischen Datensignale und der physiologischen Zieldatensignale, die bei Vorliegen des Störfaktors erzeugt werden; und
f) Bestimmen eines Werts des physiologischen Zielparameters unter Verwendung der ersten physiologischen Datensignale, der zweiten physiologischen Datensignale und der physiologischen Zieldatensignale, die bei Nichtvorliegen des Störfaktors erzeugt werden.

2. Verfahren nach Anspruch 1, wobei die ersten physiologischen Datensignale, die zweiten physiologischen Datensignale und die physiologischen Zieldatensignale, die bei Vorliegen des Störfaktors erzeugt werden, nicht in dem Schritt des Bestimmens des Werts des physiologischen Zielparameters verwendet werden.

3. Verfahren nach Anspruch 1 oder Anspruch 2, wobei:
a) die erste Kohärenz auf einem einzelnen Band von Frequenzen basiert, wobei die erste Kohärenz optional repräsentativ für Kohärenzwerte bei unterschiedlichen individuellen Frequenzen innerhalb des einzelnen Bands von Frequenzen ist; oder
b) die erste Kohärenz auf mehreren Frequenzbändern basiert, wobei die erste Kohärenz optional kollektiv repräsentativ für einen jeweiligen Kohärenzwert von jedem jeweiligen Frequenzband der mehreren Frequenzbänder ist.

4. Verfahren nach Anspruch 1 oder Anspruch 2, ferner umfassend Bestimmen eines ersten Trends des ersten physiologischen Parameters, eines zweiten Trends des zweiten physiologischen Parameters und eines dritten Trends des physiologischen Zielparameters und Vergleichen des ersten Trends, des zweiten Trends und des dritten Trends relativ zueinander,
wobei in dem Schritt des Bestimmens des Vorliegens oder Nichtvorliegens des Störfaktors optional ein oder mehrere Polaritätsfilter verwendet werden, die dazu ausgelegt sind, den ersten Trend, den zweiten Trend und den dritten Trend zu bewerten.

5. Verfahren nach einem der vorhergehenden Ansprüche, wobei:
a) in dem Schritt des Bestimmens des Vorliegens oder Nichtvorliegens des Störfaktors eine Indextabelle verwendet wird; und/oder
b) die Schritte a-f während des Zeitraums kontinuierlich durchgeführt werden.

6. Verfahren nach einem der vorhergehenden Ansprüche, wobei sich der physiologische Zielparameter auf eine Gesamthämoglobinkonzentration pro Volumen von Gewebe (THb), das erfasst wird, bezieht,
wobei der physiologische Zielparameter optional eine relative Gesamthämoglobinkonzentration pro Volumen von Gewebe (rTHb), das erfasst wird, ist und
wobei die dritte Erfassungsvorrichtung optional ein Nahinfrarotspektroskopie(NIRS)-Gewebeoximeter ist.

7. Verfahren nach Anspruch 6, wobei:
a) der erste physiologische Parameter sich auf einen Blutdruck des Subjekts bezieht und die erste Erfassungsvorrichtung eine Blutdruckerfassungsvorrichtung ist; und/oder
b) der zweite physiologische Parameter sich auf eine Herzfrequenz des Subjekts bezieht.

8. System (20) zum Bestimmen eines physiologischen Zielparameters eines Subjekts, umfassend:
eine erste Erfassungsvorrichtung (26), die dazu ausgelegt ist, einen ersten physiologischen Parameter kontinuierlich während eines Zeitraums zu erfassen und erste physiologische Datensignale zu erzeugen, die repräsentativ für den ersten physiologischen Parameter während des Zeitraums sind;
eine zweite Erfassungsvorrichtung, die dazu ausgelegt ist, einen zweiten physiologischen Parameter kontinuierlich während des Zeitraums zu erfassen und zweite physiologische Datensignale zu erzeugen, die repräsentativ für den zweiten physiologischen Parameter während des Zeitraums sind;
eine dritte Erfassungsvorrichtung (22), die dazu ausgelegt ist, einen physiologischen Zielparameter kontinuierlich während des Zeitraums zu erfassen und physiologische Zieldatensignale zu erzeugen, die repräsentativ für den physiologischen Zielparameter während des Zeitraums sind;
eine Systemsteuerung (24) in Kommunikation mit der ersten Erfassungsvorrichtung, der zweiten Erfassungsvorrichtung und der Zielerfassungsvorrichtung, wobei die Systemsteuerung mindestens einen Prozessor und eine Speichervorrichtung aufweist, die dazu ausgelegt ist, Anweisungen zu speichern, wobei die gespeicherten Anweisungen, wenn sie ausgeführt werden, bewirken, dass die Systemsteuerung Folgendes durchführt:
a) Bestimmen eines Vorliegens oder Nichtvorliegens eines Störfaktors, der eine Bestimmung des physiologischen Zielparameters verfälscht, wobei bei der Bestimmung die ersten physiologischen Datensignale, die zweiten physiologischen Datensignale und die physiologischen Zieldatensignale verwendet werden, wobei die gespeicherten Anweisungen, wenn sie ausgeführt werden, bewirken, dass die Systemsteuerung das Vorliegen oder Nichtvorliegen des Störfaktors unter Verwendung einer Frequenzbereichsmethodik bestimmt, und bewirken, dass die Systemsteuerung eine erste Kohärenz zwischen den ersten physiologischen Datensignalen und den physiologischen Zieldatensignalen und eine zweite Kohärenz zwischen den zweiten physiologischen Datensignalen und den physiologischen Zieldatensignalen bestimmt;
b) Weiterleiten der ersten physiologischen Datensignale, der zweiten physiologischen Datensignale und der physiologischen Zieldatensignale, die bei Nichtvorliegen des Störfaktors erzeugt werden, zur weiteren Verarbeitung und Zurückstellen der ersten physiologischen Datensignale, der zweiten physiologischen Datensignale und der physiologischen Zieldatensignale, die bei Vorliegen des Störfaktors erzeugt werden; und
c) Bestimmen eines Werts des physiologischen Zielparameters unter Verwendung der ersten physiologischen Datensignale, der zweiten physiologischen Datensignale und der physiologischen Zieldatensignale, die bei Nichtvorliegen des Störfaktors erzeugt werden.

9. System nach Anspruch 8, wobei die gespeicherten Anweisungen, wenn sie ausgeführt werden, bewirken, dass die Systemsteuerung den Wert des physiologischen Zielparameters bestimmt, ohne die ersten physiologischen Datensignale, die zweiten physiologischen Datensignale und die physiologischen Zieldatensignale, die bei Vorliegen des Störfaktors erzeugt werden, zu verwenden.

10. System nach Anspruch 8 oder Anspruch 9, wobei:
a) die erste Kohärenz auf einem einzelnen Band von Frequenzen basiert, wobei die erste Kohärenz optional repräsentativ für Kohärenzwerte bei unterschiedlichen individuellen Frequenzen innerhalb des einzelnen Bands von Frequenzen ist; oder
b) die erste Kohärenz auf mehreren Frequenzbändern basiert, wobei die erste Kohärenz optional kollektiv repräsentativ für einen jeweiligen Kohärenzwert von jedem jeweiligen Frequenzband der mehreren Frequenzbänder ist.

11. System nach einem der Ansprüche 8 bis 10, wobei die gespeicherten Anweisungen, wenn sie ausgeführt werden, bewirken, dass die Systemsteuerung einen ersten Trend des ersten physiologischen Parameters, einen zweiten Trend des zweiten physiologischen Parameters und einen dritten Trend des physiologischen Zielparameters bestimmt und den ersten Trend, den zweiten Trend und den dritten Trend relativ zueinander vergleicht,
wobei die gespeicherten Anweisungen, wenn sie ausgeführt werden, optional bewirken, dass die Systemsteuerung das Vorliegen oder Nichtvorliegen des Störfaktors unter Verwendung eines oder mehrerer Polaritätsfilter bestimmt, die dazu ausgelegt sind, den ersten Trend, den zweiten Trend und den dritten Trend zu bewerten.

12. System nach Anspruch 21, wobei die gespeicherten Anweisungen, wenn sie ausgeführt werden, bewirken, dass die Systemsteuerung Folgendes durchführt:
a) Bestimmen des Vorliegens oder Nichtvorliegens des Störfaktors unter Verwendung einer Indextabelle; und/oder
b) kontinuierliches Durchführen der Funktionen a-c während des Zeitraums.

13. System nach einem der Ansprüche 8 bis 12, wobei sich der physiologische Zielparameter auf eine Gesamthämoglobinkonzentration pro Volumen von Gewebe (THb), das erfasst wird, bezieht,
wobei der physiologische Zielparameter optional eine relative Gesamthämoglobinkonzentration pro Volumen von Gewebe (rTHb), das erfasst wird, ist und
wobei die dritte Erfassungsvorrichtung optional ein Nahinfrarotspektroskopie(NIRS)-Gewebeoximeter ist.

14. System nach Anspruch 13, wobei:
a) der erste physiologische Parameter sich auf einen Blutdruck des Subjekts bezieht und die erste Erfassungsvorrichtung eine Blutdruckerfassungsvorrichtung ist; und/oder
b) der zweite physiologische Parameter sich auf eine Herzfrequenz des Subjekts bezieht.

15. Nichtflüchtiges computerlesbares Medium, das ausführbare Anweisungen speichert, die, wenn sie ausgeführt werden, bewirken, dass mindestens ein Prozessor Folgendes durchführt:
Steuern einer ersten Erfassungsvorrichtung, die dazu ausgelegt ist, einen ersten physiologischen Parameter zu erfassen, um ein Subjekt zu erfassen und erste physiologische Datensignale zu erzeugen, die repräsentativ für den ersten physiologischen Parameter während eines Zeitraums sind;
Steuern einer zweiten Erfassungsvorrichtung, die dazu ausgelegt ist, einen zweiten physiologischen Parameter zu erfassen, um das Subjekt zu erfassen und zweite physiologische Datensignale zu erzeugen, die repräsentativ für den zweiten physiologischen Parameter während des Zeitraums sind;
Steuern einer dritten Erfassungsvorrichtung, die dazu ausgelegt ist, einen physiologischen Zielparameter zu erfassen, um das Subjekt zu erfassen und physiologische Zieldatensignale zu erzeugen, die repräsentativ für den physiologischen Zielparameter während des Zeitraums sind;
Bestimmen eines Vorliegens oder Nichtvorliegens eines Störfaktors, der eine Bestimmung des physiologischen Zielparameters verfälscht, wobei bei der Bestimmung die ersten physiologischen Datensignale, die zweiten physiologischen Datensignale und die physiologischen Zieldatensignale verwendet werden, wobei in dem Schritt des Bestimmens des Vorliegens oder Nichtvorliegens des Störfaktors eine Frequenzbereichsmethodik verwendet wird und wobei der Schritt des Bestimmens des Vorliegens oder Nichtvorliegens des Störfaktors Bestimmen einer ersten Kohärenz zwischen den ersten physiologischen Datensignalen und den physiologischen Zieldatensignalen und einer zweiten Kohärenz zwischen den zweiten physiologischen Datensignalen und den physiologischen Zieldatensignalen beinhaltet;
Weiterleiten der ersten physiologischen Datensignale, der zweiten physiologischen Datensignale und der physiologischen Zieldatensignale, die bei Nichtvorliegen des Störfaktors erzeugt werden, zur weiteren Verarbeitung und Zurückstellen der ersten physiologischen Datensignale, der zweiten physiologischen Datensignale und der physiologischen Zieldatensignale, die bei Vorliegen des Störfaktors erzeugt werden; und
Bestimmen eines Werts des physiologischen Zielparameters unter Verwendung der ersten physiologischen Datensignale, der zweiten physiologischen Datensignale und der physiologischen Zieldatensignale, die bei Nichtvorliegen des Störfaktors erzeugt werden.

## Revendications

1. Procédé de détermination d'un paramètre physiologique cible d'un sujet, comprenant les étapes consistant à :
a) détecter un sujet avec un premier dispositif de détection configuré pour détecter un premier paramètre physiologique, le premier dispositif de détection produisant des premiers signaux de données physiologiques représentatifs du premier paramètre physiologique pendant une certaine période de temps ;
b) détecter le sujet avec un deuxième dispositif de détection configuré pour détecter un deuxième paramètre physiologique, le deuxième dispositif de détection produisant des deuxièmes signaux de données physiologiques représentatifs du deuxième paramètre physiologique pendant la période de temps ;
c) détecter le sujet avec un troisième dispositif de détection configuré pour détecter un paramètre physiologique cible, le troisième dispositif de détection produisant des signaux de données physiologiques cibles représentatifs du paramètre physiologique cible pendant la période de temps ;
d) déterminer une présence ou une absence d'un facteur de confusion qui entache une détermination du paramètre physiologique cible, la détermination utilisant les premiers signaux de données physiologiques, les deuxièmes signaux de données physiologiques et les signaux de données physiologiques cibles, l'étape de détermination de la présence ou de l'absence du facteur de confusion utilisant une méthodologie de domaine fréquentiel, et l'étape de détermination de la présence ou de l'absence du facteur de confusion comprenant la détermination d'une première cohérence entre les premiers signaux de données physiologiques et les signaux de données physiologiques cibles, et d'une deuxième cohérence entre les deuxièmes signaux de données physiologiques et les signaux de données physiologiques cibles ;
e) avancer les premiers signaux de données physiologiques, les deuxièmes signaux de données physiologiques et les signaux de données physiologiques cibles produits en l'absence du facteur de confusion pour un traitement ultérieur, et mettre de côté les premiers signaux de données physiologiques, les deuxièmes signaux de données physiologiques et les signaux de données physiologiques cibles produits en présence du facteur de confusion ; et
f) déterminer une valeur du paramètre physiologique cible à l'aide des premiers signaux de données physiologiques, des deuxièmes signaux de données physiologiques et des signaux de données physiologiques cibles produits en l'absence du facteur de confusion.

2. Procédé selon la revendication 1, dans lequel les premiers signaux de données physiologiques, les deuxièmes signaux de données physiologiques et les signaux de données physiologiques cibles produits en présence du facteur de confusion ne sont pas utilisés dans l'étape de détermination de la valeur du paramètre physiologique cible.

3. Procédé selon la revendication 1 ou la revendication 2, dans lequel :
a) la première cohérence est basée sur une bande de fréquences unique, optionnellement la première cohérence étant représentative de valeurs de cohérence à différentes fréquences individuelles à l'intérieur de la bande de fréquences unique ; ou
b) la première cohérence est basée sur une pluralité de bandes de fréquence, optionnellement la première cohérence étant collectivement représentative d'une valeur de cohérence respective issue de chaque bande de fréquences respective de la pluralité de bandes de fréquence.

4. Procédé selon la revendication 1 ou la revendication 2, comprenant en outre la détermination d'une première tendance du premier paramètre physiologique, d'une deuxième tendance du deuxième paramètre physiologique et d'une troisième tendance du paramètre physiologique cible, et la comparaison de la première tendance, de la deuxième tendance et de la troisième tendance les unes par rapport aux autres.
dans lequel, optionnellement, l'étape de détermination de la présence ou de l'absence du facteur de confusion utilise un ou plusieurs filtres de polarité configurés pour évaluer la première tendance, la deuxième tendance et la troisième tendance.

5. Procédé selon l'une quelconque des revendications précédentes, dans lequel :
a) l'étape de détermination de la présence ou de l'absence du facteur de confusion utilise une table d'index ; et/ou
b) les étapes a à f sont effectuées de manière continue pendant la période de temps.

6. Procédé selon l'une quelconque des revendications précédentes, dans lequel le paramètre physiologique cible se rapporte à la concentration totale en hémoglobine par volume de tissu (THb) détectée,
dans lequel, optionnellement, le paramètre physiologique cible est la concentration totale relative d'hémoglobine par volume de tissu (rTHb) du tissu détecté, et
dans lequel, optionnellement, le troisième dispositif de détection est un oxymètre tissulaire de spectroscopie en proche infrarouge (NIRS).

7. Procédé selon la revendication 6, dans lequel :
a) le premier paramètre physiologique se rapporte à la pression sanguine du sujet, et le premier dispositif de détection est un dispositif de détection de la pression sanguine ; et/ou
b) le deuxième paramètre physiologique se rapporte à une fréquence cardiaque du sujet.

8. Système (20) de détermination d'un paramètre physiologique cible d'un sujet, comprenant :
un premier dispositif de détection (26) configuré pour détecter un premier paramètre physiologique en continu pendant une période de temps, et pour produire des premiers signaux de données physiologiques représentatifs du premier paramètre physiologique pendant la période de temps ;
un deuxième dispositif de détection configuré pour détecter un deuxième paramètre physiologique en continu pendant la période de temps, et pour produire des deuxièmes signaux de données physiologiques représentatifs du deuxième paramètre physiologique pendant la période de temps ;
un troisième dispositif de détection (22) configuré pour détecter un paramètre physiologique cible en continu pendant la période de temps, et pour produire des signaux de données physiologiques cibles représentatifs du paramètre physiologique cible pendant la période de temps ;
un dispositif de commande de système (24) en communication avec le premier dispositif de détection, le deuxième dispositif de détection et le dispositif de détection cible, le dispositif de commande de système comportant au moins un processeur et un dispositif de mémoire configuré pour stocker des instructions, les instructions stockées, lorsqu'elles sont exécutées, amenant le dispositif de commande de système à :
a) déterminer une présence ou une absence d'un facteur de confusion qui entache une détermination du paramètre physiologique cible, la détermination utilisant les premiers signaux de données physiologiques, les deuxièmes signaux de données physiologiques et les signaux de données physiologiques cibles, les instructions stockées, lorsqu'elles sont exécutées, amenant le dispositif de commande de système à déterminer la présence ou l'absence du facteur de confusion en utilisant une méthodologie de domaine fréquentiel, et amenant le dispositif de commande de système à déterminer une première cohérence entre les premiers signaux de données physiologiques et les signaux de données physiologiques cibles, et une deuxième cohérence entre les deuxièmes signaux de données physiologiques et les signaux de données physiologiques cibles ;
b) avancer les premiers signaux de données physiologiques, les deuxièmes signaux de données physiologiques et les signaux de données physiologiques cibles produits en l'absence du facteur de confusion pour un traitement ultérieur, et mettre de côté les premiers signaux de données physiologiques, les deuxièmes signaux de données physiologiques et les signaux de données physiologiques cibles produits en présence du facteur de confusion ; et
c) déterminer une valeur du paramètre physiologique cible à l'aide des premiers signaux de données physiologiques, des deuxièmes signaux de données physiologiques et des signaux de données physiologiques cibles produits en l'absence du facteur de confusion.

9. Système selon la revendication 8, dans lequel les instructions mémorisées, lorsqu'elles sont exécutées, amènent le dispositif de commande de système à déterminer la valeur du paramètre physiologique cible sans utiliser les premiers signaux de données physiologiques, les deuxièmes signaux de données physiologiques et les signaux de données physiologiques cibles produits en présence du facteur de confusion.

10. Système selon la revendication 8 ou la revendication 9, dans lequel :
a) la première cohérence est basée sur une bande de fréquences unique, optionnellement la première cohérence étant représentative de valeurs de cohérence à différentes fréquences individuelles à l'intérieur de la bande de fréquences unique ; ou
b) la première cohérence est basée sur une pluralité de bandes de fréquence, optionnellement la première cohérence étant collectivement représentative d'une valeur de cohérence respective issue de chaque bande de fréquences respective de la pluralité de bandes de fréquence.

11. Système selon l'une quelconque des revendications 8 à 10, dans lequel les instructions stockées, lorsqu'elles sont exécutées, amènent le dispositif de commande de système à déterminer une première tendance du premier paramètre physiologique, une deuxième tendance du deuxième paramètre physiologique, et une troisième tendance du paramètre physiologique cible, et à comparer la première tendance, la deuxième tendance et la troisième tendance les unes par rapport aux autres,
optionnellement, les instructions stockées, lorsqu'elles sont exécutées, amenant le dispositif de commande de système à déterminer la présence ou l'absence du facteur de confusion au moyen d'un ou de plusieurs filtres de polarité configurés pour évaluer la première tendance, la deuxième tendance et la troisième tendance.

12. Appareil selon la revendication 21, dans lequel les instructions stockées, lorsqu'elles sont exécutées, amènent le dispositif de commande de système à :
a) déterminer la présence ou l'absence du facteur de confusion à l'aide d'une table d'index ; et/ou
b) exécuter les fonctions a à c de manière continue pendant la période de temps.

13. Système selon l'une quelconque des revendications 8 à 12, dans lequel le paramètre physiologique cible se rapporte à la concentration totale en hémoglobine par volume de tissu (THB) détectée,
dans lequel, optionnellement, le paramètre physiologique cible est la concentration totale relative d'hémoglobine par volume de tissu (rTHb) du tissu détecté, et
dans lequel, optionnellement, le troisième dispositif de détection est un oxymètre tissulaire de spectroscopie en proche infrarouge (NIRS).

14. Système selon la revendication 13, dans lequel :
a) le premier paramètre physiologique se rapporte à la pression sanguine du sujet, et le premier dispositif de détection est un dispositif de détection de la pression sanguine ; et/ou
b) le deuxième paramètre physiologique se rapporte à une fréquence cardiaque du sujet.

15. Support non transitoire lisible par ordinateur stockant des instructions exécutables qui, lorsqu'elles sont exécutées, amènent au moins un processeur à :
commander un premier dispositif de détection configuré pour détecter un premier paramètre physiologique pour détecter un sujet et pour produire des premiers signaux de données physiologiques représentatifs du premier paramètre physiologique pendant une période de temps ;
commander un deuxième dispositif de détection configuré pour détecter un deuxième paramètre physiologique pour détecter le sujet et pour produire des deuxièmes signaux de données physiologiques représentatifs du deuxième paramètre physiologique pendant la période de temps ;
commander un troisième dispositif de détection configuré pour détecter un paramètre physiologique cible pour détecter le sujet et pour produire des signaux de données physiologiques cibles représentatifs du paramètre physiologique cible pendant la période de temps ;
déterminer une présence ou une absence d'un facteur de confusion qui entache une détermination du paramètre physiologique cible, la détermination utilisant les premiers signaux de données physiologiques, les deuxièmes signaux de données physiologiques et les signaux de données physiologiques cibles, l'étape de détermination de la présence ou de l'absence du facteur de confusion utilisant une méthodologie de domaine fréquentiel et l'étape de détermination de la présence ou de l'absence du facteur de confusion comprenant la détermination d'une première cohérence entre les premiers signaux de données physiologiques et les signaux de données physiologiques cibles, et d'une deuxième cohérence entre les deuxièmes signaux de données physiologiques et les signaux de données physiologiques cibles ;
avancer les premiers signaux de données physiologiques, les deuxièmes signaux de données physiologiques et les signaux de données physiologiques cibles produits en l'absence du facteur de confusion pour un traitement ultérieur, et mettre de côté les premiers signaux de données physiologiques, les deuxièmes signaux de données physiologiques et les signaux de données physiologiques cibles produits en présence du facteur de confusion ; et
déterminer une valeur du paramètre physiologique cible à l'aide des premiers signaux de données physiologiques, des deuxièmes signaux de données physiologiques et des signaux de données physiologiques cibles produits en l'absence du facteur de confusion.
